## Europäisches Patentamt
## European Patent Office
## Office européen des brevets

(19)

(11) Veröffentlichungsnummer: **0 009 630**
**B1**

(12) **EUROPÄISCHE PATENTSCHRIFT**

(45) Veröffentlichungstag der Patentschrift:
16.09.81

(21) Anmeldenummer: 79103216.2

(22) Anmeldetag: 31.08.79

(51) Int. Cl.³: **C 07 C 11/167**, C 07 C 7/00, C 07 C 7/08, C 07 C 7/04

(54) Verfahren zur Gewinnung von Butadien aus einem C4-Kohlenwasserstoff-Gemisch.

(30) Priorität: 15.09.78 DE 2840124

(43) Veröffentlichungstag der Anmeldung:
16.04.80 Patentblatt 80/8

(45) Bekanntmachung des Hinweises auf die Patenterteilung:
16.09.81 Patentblatt 81/37

(84) Benannte Vertragsstaaten:
BE DE FR GB IT NL

(56) Entgegenhaltungen:
DE-A-2 251 051
DE-A-2 742 148
US-A-2 920 113

(73) Patentinhaber: BASF Aktiengesellschaft,
Carl-Bosch-Strasse 38, D-6700 Ludwigshafen (DE)

(72) Erfinder: Lindner, Alfred, Dr., Ringstrasse 22,
D-6712 Bobenheim-Roxheim 1 (DE)
Erfinder: Volkamer, Klaus, Dr., Heidelberger Ring 21,
D-6710 Frankenthal (DE)
Erfinder: Wagner, Ulrich, Dr., Knospstrasse 7,
D-6703 Limburgerhof (DE)
Erfinder: Pommer, Dieter, Dr., Im Langental 11,
D-6719 Weisenheim (DE)
Erfinder: Schneider, Klaus-Juergen, Dr.,
Triftbrunnenweg 16, D-6730 Neustadt 19 (DE)
Erfinder: Schwentker, Harald, Dr., Osloer Weg 44,
D-6700 Ludwigshafen (DE)

EP 0 009 630 B1

## Verfahren zur Gewinnung von Butadien aus einem $C_4$-Kohlenwasserstoff-Gemisch

Die vorliegende Erfindung betrifft ein Verfahren zur Gewinnung von Butadien aus einer Mischung aus Styrol und einem $C_4$-Kohlenwasserstoffgemisch.

Es ist bekannt, z. B. aus der DE-OS 22 51 051 und DE-OS 27 42 148 Butadien aus Butadien enthaltenden $C_4$-Kohlenwasserstoffgemischen durch extraktive Destillation unter Verwendung eines selektiven Lösungsmittels zu gewinnen. Gemäss dem Verfahren der DE-OS 22 51 051 wird dabei das $C_4$-Kohlenwasserstoffgemisch, das für die extraktive Destillation eingesetzt wird, durch Destillation eines aus einer Ethylenanlage erhaltenen Kohlenwasserstoffstromes erhalten, indem in einer ersten Destillationskolonne als Kopfprodukt die in dem Kohlenwasserstoffstrom aus der Ethylenanlage enthaltenen $C_3$-Kohlenwasserstoffe und in einer zweiten Destillationskolonne als Sumpfprodukt die $C_5$-Kohlenwasserstoffe abgetrennt werden, wobei ein nur noch sehr geringe Mengen $C_3$- und/oder $C_5$-Kohlenwasserstoffe enthaltendes $C_4$-Kohlenwasserstoffgemisch erhalten wird. Auch gemäss dem Verfahren der DE-OS 27 42 148 wird für die extraktive Destillation ein geringe Mengen $C_5$-Kohlenwasserstoffe enthaltendes $C_4$-Kohlenwasserstoffgemisch eingesetzt, welches im allgemeinen ebenfalls durch Fraktionierung aus einem aus einer Ethylenanlage erhaltenen Kohlenwasserstoffstrom erhalten worden ist. Das nach der extraktiven Destillation erhaltene Butadien wird im allgemeinen anschliessend noch einer konventionellen Destillation unterworfen.

Ein wichtiger Verwendungszweck des durch extraktive Destillation erhaltenen Butadiens ist die Herstellung von Butadien-Styrol-Copolymerisaten, wobei nach der Polymerisation ein Styrol enthaltender Butadien-Abfallstrom erhalten wird. Dieser Abfallstrom kann beispielsweise verbrannt werden, wird jedoch zweckmässigerweise in eine Butadienextraktionsanlage zurückgeführt. Durch Initiierung der Polymerisation mit Sauerstoff kann der Styrol enthaltende Butadien-Abfallstrom noch geringe Mengen Sauerstoff enthalten.

Eine direkte Rückführung des geringe Mengen Styrol und gegebenenfalls Sauerstoff enthaltenden Butadien-Abfallstromes in eine Butadienextraktionsanlage würde innerhalb der Butadienextraktionsanlage zu unerwünschten Polymerbildungen führen. Hierdurch würden die Laufzeiten der Anlage erheblich verringert.

Aus der US-Patentschrift 2 920 113 ist es bereits bekannt, dass man zur Rückgewinnung von Butadien aus Butadien/Styrol-Gemischen die Gemische fraktioniert, wobei Butadien als Kopfprodukt und eine Mischung aus Styrol und Butadien als Sumpfprodukt erhalten wird. Das als Kopfprodukt erhaltene Butadien wird anschliessend noch weiter gereinigt. Bei dieser Arbeitsweise ist es von Nachteil, dass durch das im Sumpfprodukt der Fraktionierung abgezogene Butadien ein erheblicher Butadienverlust auftritt.

Es wurde nun ein vorteilhaftes Verfahren gefunden zur Gewinnung von Butadien-1,3 mit Hilfe eines selektiven Lösungsmittels aus einer Mischung aus Styrol und einem $C_4$-Kohlenwasserstoffgemisch, welches Butadien-1,3 im selektiven Lösungsmittel löslichere Kohlenwasserstoffe als Butadien-1,3 und im selektiven Lösungsmittel weniger lösliche Kohlenwasserstoffe als Butadien-1,3 enthält, wobei die Mischung gegebenenfalls zusätzlich geringere Mengen Sauerstoff enthält, welches dadurch gekennzeichnet ist, dass man die Mischung zunächst einer Destillationszone zuführt, wobei der Destillationszone gleichzeitig ein zweiter Strom von flüssigen oder gasförmigen Kohlenwasserstoffen zugeführt wird, aus der Destillationszone als Sumpfprodukt ein Gemisch aus Styrol und $C_4$-Kohlenwasserstoffen abtrennt und als Kopfprodukt ein $C_4$-Kohlenwasserstoffgemisch erhält und anschliessend das als Kopfprodukt der Destillationszone erhaltene $C_4$-Kohlenwasserstoffgemisch einer extraktiven Destillation zuführt, in der das $C_4$-Kohlenwasserstoffgemisch in ein die weniger löslichen Kohlenwasserstoffe enthaltendes Destillat, einen Strom aus Butadien-1,3 und einen die löslicheren Kohlenwasserstoffe enthaltenden Strom aufgetrennt wird.

Nach dem neuen Verfahren ist es möglich, Butadien aus $C_4$-Kohlenwasserstoffgemischen zu gewinnen, ohne Styrol in die Butadienlage einschleppen zu müssen, so dass eine kontinuierliche Durchführung des Verfahrens über ausgedehnte Zeiträume möglich wird. Es ist ein besonderer Vorteil dieses Verfahrens, dass der mit der Abtrennung des Gemisches aus Styrol und $C_4$-Kohlenwasserstoffen in der Destillationszone verbundene Butadien-Verlust besonders niedrig gehalten werden kann.

Die als Ausgangsstoff verwendete Mischung aus Styrol und dem $C_4$-Kohlenwasserstoffgemisch wird beispielsweise bei der Herstellung von Butadien-Styrol-Copolymerisaten erhalten. Eine solche Mischung kann neben Butadien und Styrol noch Buten-2-cis, Buten-2-trans, Butadien-1,2, Butan, iso-Butan, Buten-1, Butin-1, Butenin sowie $C_3$-Kohlenwasserstoffe wie Propan, Propen, Propadien und Propin enthalten. Im allgemeinen weisen die Ausgangskohlenwasserstoffgemische Styrolgehalte von 0,05 bis 10 Gew.-%, vorzugsweise 0,1 bis 5 Gew.-%, auf.

Erfindungsgemäss wird aus dem Styrol enthaltenden Ausgangskohlenwasserstoffgemisch in einer der extraktiven Destillation vorgeschalteten Destillationszone ein Gemisch aus Styrol und $C_4$-Kohlenwasserstoffen abgetrennt und das Kopfprodukt der vorgeschalteten Destillationszone der extraktiven Destillation zugeführt. Im allgemeinen weist das abgetrennte Gemisch aus Styrol und den $C_4$-Kohlenwasserstoffen einen Styrol-Gehalt von 0,5 bis 30 Gew.-%, vorzugsweise von 1 bis 20 Gew.-%, insbesondere 2 bis 15 Gew.-% auf, wobei sich die obere Begrenzung des Styrolgehaltes in allgemeinen aus der Begrenzung der Sumpftemperatur ergibt. Um die Polymerisationsneigung des Styrols und des Butadiens gering zu halten, wird in der vorgeschalteten Destillationszone zweckmässig eine möglichst niedrige Sumpftemperatur eingestellt. Im allgemeinen liegt die Sumpftemperatur zwischen 40 und 70 °C, vorzugsweise zwischen 45 und 50 °C.

Andererseits ist es vorteilhaft, den Rücklauf der Kolonne durch Kondensation der $C_4$-Kohlenwasserstoffe mit Kühlwasser zu erzeugen. Dies hätte bei einer Arbeitsweise ohne Zuführung eines zweiten Stromes von flüssigen oder gasförmigen Kohlenwasserstoffen einen hohen Gehalt des Sumpfproduktes an $C_4$-Kohlenwasserstoffen und somit einen erheblichen Butadien-Verlust zur Folge. Durch die erfindungsgemässe Zuführung eines zweiten Stromes von flüssigen oder gasförmigen Kohlenwasserstoffen zur Destillationszone, die ein wesentliches Merkmal des Verfahrens der vorliegenden Erfindung darstellt, kann nun dieser mit der Abtrennung des Gemisches aus Styrol und $C_4$-Kohlenwasserstoffen verbundene Butadien-Verlust sehr gering gehalten werden.

Als flüssige oder gasförmige Kohlenwasserstoffe, die der Destillationszone als zweiter Strom zugeführt werden, werden im allgemeinen $C_3$- und/oder $C_4$-Kohlenwasserstoffe eingesetzt. Zweckmässigerweise werden Kohlenwasserstoffe mit einem geringeren Butadiengehalt als das Ausgangs-$C_4$-Kohlenwasserstoffgemisch, z. B. mit weniger als 90% des Butadiengehalts des Ausgangs-$C_4$-Kohlenwasserstoffgemisches, vorzugsweise im wesentlichen Butadien-freie Kohlenwasserstoffe, z. B. mit einem Butadiengehalt von weniger als 5 Gew.-%, zweckmässig weniger als 1 Gew.-%, eingesetzt. Vorteilhaft werden gesättigte und/oder einfach olefinisch ungesättigte $C_3$- und/oder $C_4$-Kohlenwasserstoffe verwendet. Geeignete Kohlenwasserstoffe sind z. B. Propan, Propen, die Butane, n-Buten, Isobuten, die 2-Butene, Butadien-1,2 oder diese Kohlenwasserstoffe enthaltende Gemische. Vorteilhaft werden $C_4$-Kohlenwasserstoffgemische eingesetzt. In einer bevorzugten Ausführungsform des Verfahrens wird das in der extraktiven Destillation erhaltene Raffinat verwendet, welches die Butane und Butene enthält.

Im allgemeinen wird der der vorgeschalteten Destillationszone zuzuführende zweite Kohlenwasserstrom am oder zweckmässig unterhalb des Zulaufpunktes des Styrol enthaltenden Ausgangskohlenwasserstoffgemisches der vorgeschalteten Destillationszone zugeführt, wobei eine Zuführung im unteren Drittel, vorteilhaft im unteren Viertel, insbesondere am Sumpf der vorgeschalteten Destillationszone, bevorzugt wird. Durch die Zuführung des zweiten Kohlenwasserstoffstromes unterhalb des Zulaufpunktes des Ausgangskohlenwasserstoffgemisches wird durch die dadurch erfolgende Gegenstromwäsche als Sumpfprodukt ein Gemisch aus Styrol und $C_4$-Kohlenwasserstoffen mit einem geringen Butadiengehalt, z. B. von weniger als 30 Gew.-%, vorzugsweise weniger als 5 Gew.-% Butadien, abgetrennt, so dass durch diese Arbeitsweise der mit der Styrol-Abtrennung verbundene Butadienverlust besonders niedrig gehalten werden kann. In einer bevorzugten Ausführungsform des erfindungsgemässen Verfahrens wird der zweite Kohlenwasserstoffstrom dem vom Sumpf aufsteigenden Dampf der vorgeschalteten Destillationszone zugemischt oder als Flüssigkeit dem Sumpf zugeführt.

Das erfindungsgemäss zu verwendende Ausgangswasserstoffgemisch kann neben Styrol auch noch geringe Mengen Sauerstoff enthalten. In diesem Falle wird das Styrol und geringe Mengen Sauerstoff enthaltende Ausgangskohlenwasserstoffgemisch zweckmässig zunächst einer Destillationszone für die Sauerstoffabtrennung zugeführt, in der ein den Sauerstoff enthaltendes Kopfprodukt und ein das Styrol enthaltendes Sumpfprodukt enthalten werden. Das Styrol enthaltende Sumpfprodukt wird danach als Einsatzstoff für die der extraktiven Destillation vorgeschaltete Destillationszone verwendet.

Die Destillationszone für die Sauerstoffabtrennung kann mit einem eigenen Aufkocher versehen sein, um den für die Trennung erforderlichen Dampf zu erzeugen. In einer bevorzugten Ausführungsform des erfindungsgemässen Verfahrens wird ein Teil des Kopfproduktes der der extraktiven Destillation vorgeschalteten Destillationszone, im allgemeinen 5 bis 50%, vorzugsweise 10 bis 20% des Kopfproduktes, am Sumpf der Destillationszone für die Sauerstoffabtrennung zugeführt, vorzugsweise als gasförmiger Strom.

Beide Destillationszonen können baulich getrennt in zwei Kolonnen oder baulich vereinigt in einer Kolonne geschaltet werden. Bevorzugt wird die bauliche Vereinigung der Kolonnen, wobei der Sumpf der Destillationszone für die Sauerstoffabtrennung als Abzugstasse ausgebildet werden kann. Durch diese Massnahmen können die Investitionskosten vermindert werden, da die Sumpfpumpe und der Aufkocher der Destillationszone für die Sauerstoffabtrennung entfallen können und für beide Destillationszonen nur eine Kolonne erforderlich ist.

Das Kopfprodukt aus der der extraktiven Destillation vorgeschalteten Destillationszone kann ohne Vermischung mit anderen Butadien enthaltenden $C_4$-Kohlenwasserstoffgemischen der extraktiven Destillation zugeführt werden. Zweckmässig wird das Kopfprodukt jedoch vor der Zuführung zur extraktiven Destillation einem Butadien enthaltenden $C_4$-Kohlenwasserstoffgemisch zugeführt, der den Hauptstrom zur extraktiven Destillation darstellt. Solche als Hauptstrom zur extraktiven Destillation zu verwendenden Butadien enthaltenden $C_4$-Kohlenwasserstoffgemische werden z. B. bei der Herstellung von Äthylen und/oder Propylen durch thermisches Spalten einer Petroleumfraktion, z. B. von verflüssigtem Petroleumgas (LPG), Leichtbenzin (Naphtha), Gasöl und dergleichen, als Kohlenwasserstofffraktion erhalten. Weiterhin werden solche C₄-Fraktionen bei der katalytischen Dehydrierung von n-Butan und/oder n-Buten erhalten.

Als selektives Lösungsmittel kommen beispielsweise Carbonsäureamide, wie Dimethylformamid, Diäthylformamid, Dimethylacetamid, Formylmorpholin, Acetonitril, Furfurol, N-Methylpyrrolidon, Butyrolacton, Aceton und ihre Mischungen mit Wasser in Betracht. Mit besonderem Vorteil wird N-Methylpyrrolidon als selektives Lösungsmittel verwendet. Im selektiven Lösungsmittel löslichere Kohlenwasserstoffe als Butadien sind beispielsweise Vinylacetylen, Äthylacetylen, Butadien-1,2. Im selektiven Lösungsmittel weniger lösliche Kohlenwasserstoffe als Butadien sind beispielsweise die Butane, die n-Butene, Isobuten. Die extraktive Destillation kann unter Anwendung einer Extraktivdestillationszone durchgeführt werden. Mit besonderem Vorteil wird das Verfahren zur Gewinnung von Butadien jedoch unter Anwendung von zwei hintereinandergeschalteten Extraktivdestillationszonen unter Verwendung des gleichen selektiven Lösungsmittels durchgeführt. Hierbei wird beispielsweise in der ersten Stufe der Extraktivdestillation ein die weniger löslichen Kohlenwasserstoffe enthaltendes Destillat (Raffinat) und ein Butadien und die löslicheren Kohlenwasserstoffe und das selektive Lösungsmittel enthaltender Extrakt erhalten. Dieser Extrakt wird vom selektiven Lösungsmittel befreit wobei ein Gemisch aus Butadien und den löslicheren Kohlenwasserstoffen erhalten wird. Dieses Gemisch wird in einer zweiten extraktiven Destillationszone einer zweiten extraktiven Destillation mit dem selektiven Lösungsmittel unterworfen, wobei Butadien als Destillat und ein Extrakt erhalten werden, der die löslicheren Kohlenwasserstoffe einschliesslich der höheren Acetylene und noch zurückgebliebenes Butadien und das selektive Lösungsmittel enthält. Der erhaltene Extrakt wird anschliessend vom selektiven Lösungsmittel befreit, wobei ein die löslicheren Kohlenwasserstoffe einschliesslich der C₄-Acetylene enthaltender Kohlenwasserstoffstrom erhalten wird.

Das aus der extraktiven Destillation erhaltene Butadien wird im allgemeinen zur Entfernung von noch gegebenenfalls vorhandenen geringen Mengen von Propin und C₅-Kohlenwasserstoffen einer nachgeschalteten Destillation, z. B. in einer oder zwei Destillationskolonnen, unterworfen. Die destillative Abtrennung von Propin und C₅-Kohlenwasserstoffen kann jedoch auch vor der Extraktivdestillation durchgeführt werden.

Es kann vorteilhaft sein, zur Vermeidung von unerwünschten Polymerbildungen zusätzlich zu der Styrol-Abtrennung aus dem Ausgangskohlenwasserstoffgemisch in der vorgeschalteten Destillationszone bei der anschliessenden extraktiven Destillation in an sich bekannter Weise Polymerisationsinhibitoren zuzugeben. Geeignete Polymerisationsinhibitoren sind z. B. Furfurol, Benzaldehyd, aliphatische oder aromatische Nitroverbindungen, Hydrochinon, Schwefel, Natriumnitrit, phenolische Verbindungen, wie 4-tert.-Butylcatechol und aromatische Amine, wie Naphthylamin. Hierdurch kann die Polymerisationsneigung des Butadiens zusätzlich vermindert werden. Diese Inhibitoren können ebenso in der Destillationszone zur Styrolabtrennung eingesetzt werden.

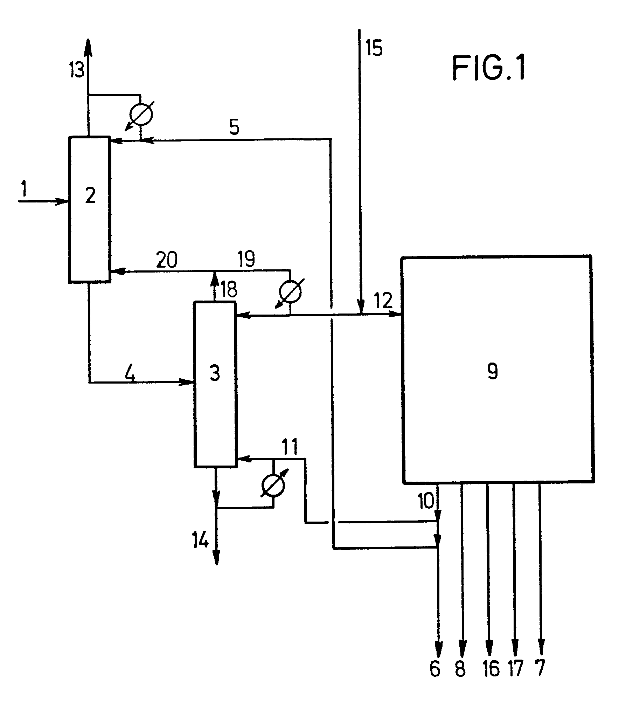

Figur 1 ist eine Ausführungsform des erfindungsgemässen Verfahrens. Eine geringe Menge Sauerstoff enthaltende Mischung aus Styrol und einem C₄-Kohlenwasserstoffgemisch wird durch Leitung 1 in das mittlere Drittel der Destillationszone 2 eingeführt. Am Kopf der Kolonne 2 wird durch Leitung 5 ein Teilstrom des Raffinats einer nachgeschalteten Butadien-Gewinnungsanlage 9 zugeführt. Vom Sumpf der Destillationszone 2 wird durch Leitung 4 sauerstofffreies Kohlenwasserstoffgemisch in das mittlere Drittel der Destillationszone 3 eingeleitet. Am Kopf der Destillationszone 2 wird sauerstoffhaltiges C₄-Kohlenwasserstoffgemisch 13 abgezogen. Am Sumpf der Destillationszone 3 wird Raffinat aus einer nachgeschalteten Butadien-Gewinnungsanlage durch Leitung 11 zugeführt. Durch Leitung 14 wird am Sumpf der Destillationszone 3 styrolhaltiges C₄-Kohlenwasserstoffgemisch mit geringem Butadiengehalt abgezogen. Am Kopf der Destillationszone 3 wird (über Leitungen 18 und 19) styrol- und sauerstofffreies Gemisch abgezogen, gegebenenfalls mit anderen C₄-Kohlenwasserstoffgemischen (über Leitung 15) vermischt und der Butadienanlage durch Leitung 12 zugeführt, in der es durch extraktive Destillation unter Verwendung eines selektiven Lösungsmittels (N-Methylpyrrolidon) in eine die Butene enthaltende Fraktion (Raffinat) 10, einen Rohbutadienstrom und den die C₄-Acetylene enthaltenden Strom 8 aufgetrennt wird. Der Rohbutadienstrom und die darin enthaltenen C₃- und C₅-Kohlenwasserstoffe sowie Butadien-1,2 werden destillativ in den Propinstrom 16, den Butadien-1,2-Strom 17 und den Reinbutadienstrom 7 aufgetrennt. Der nach Abzweigen der Ströme 5 und 11 verbleibende Teilstrom des Raffinats wird über Leitung 6 abgezogen. Ein Teil des Kopfproduktes der Destillationszone 3 wird über Leitungen 18 und 20 dem Sumpf der Destillationszone 2 zugeführt.

Destillationszonen 2 und 3 können als baulich getrennte Kolonnen, wie in Fig. 1 angegeben, oder baulich vereinigt werden, wobei der Sumpf der Destillationszone 2 als Abzugstasse ausgebildet werden kann (Fig. 2). Das Verfahren kann jedoch auch wie in Fig. 3 dargestellt mit zwei getrennten, konventionell verschalteten Kolonnen, d. h. ohne Rückführung von Kopfprodukt aus Destillationszone 3 in Destillationszone 2, ausgeführt sein.

Das nachstehende Beispiel dient der weiteren Erläuterung der Erfindung.

Beispiel

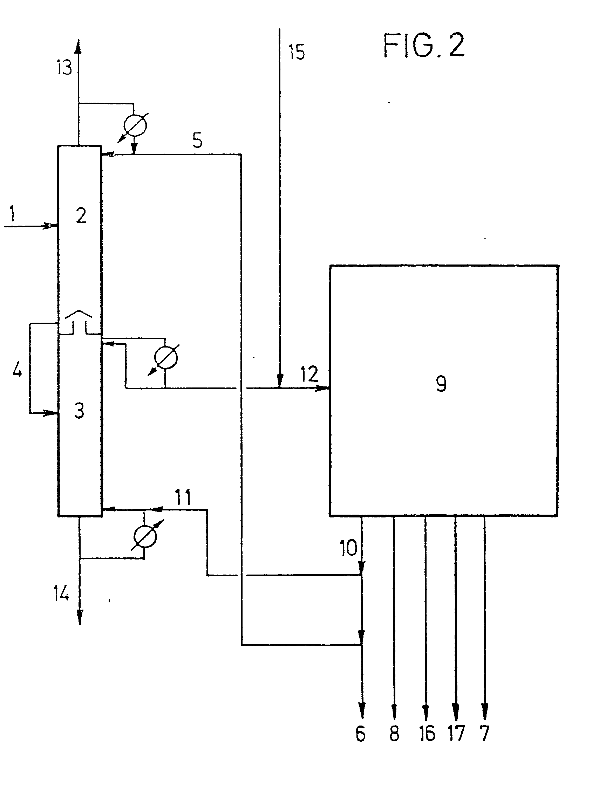

Ein Kohlenwasserstoffgemisch der folgenden Zusammensetzung wird in einer Apparatur gemäss Fig. 2 aufgetrennt:

kennzeichnet, dass ein Teil des Kopfproduktes der der extraktiven Destillation vorgeschalteten Destillationszone am Sumpf der Destillationszone für die Sauerstoffabtrennung zugeführt wird.

7. Verfahren nach Anspruch 1 bis 6, dadurch gekennzeichnet, dass das Kopfprodukt aus der der extraktiven Destillation vorgeschalteten Destillationszone vor der Zuführung zur extraktiven Destillation einem Butadien enthaltenden $C_4$-Kohlenwasserstoffgemisch zugeführt wird, das den Hauptstrom zur extraktiven Destillation darstellt.

## Claims

1. A process for isolating 1,3-butadiene, with the aid of a selective solvent, from a mixture of styrene and a $C_4$-hydrocarbon mixture containing 1,3-butadiene and hydrocarbons more soluble than 1,3-butadiene in the selective solvent, and hydrocarbons less soluble than 1,3-butadiene in the selective solvent, it being possible for the mixture to additionally contain small amounts of oxygen, characterized in that the mixture is first fed to a distillation zone, a second stream of liquid or gaseous hydrocarbons being simultaneously fed to the distillation zone, a mixture of styrene and $C_4$-hydrocarbons is separated from the distillation zone as bottom product and a $C_4$-hydrocarbon mixture is obtained as top product, and then the $C_4$-hydrocarbon mixture obtained as top product of the distillation zone is fed to an extractive distillation where the $C_4$-hydrocarbon mixture is separated into a distillate containing the less soluble hydrocarbons, a stream of 1,3-butadiene and a stream containing the more soluble hydrocarbons.

2. A process as claimed in claim 1, characterized in that the second stream fed to the distillation zone is a saturated or monoolefinically unsaturated $C_4$-hydrocarbon or a mixture of saturated and/or monoolefinically unsaturated $C_4$-hydrocarbons.

3. A process as claimed in claims 1 and 2, characterized in that the second hydrocarbon stream is introduced into the distillation zone at or below the feed point of the mixture of styrene and the $C_4$-hydrocarbon mixture.

4. A process as claimed in claims 1 to 3, characterized in that the second hydrocarbon stream is introduced into the lower third of the distillation zone.

5. A process as claimed in claims 1 to 4, characterized in that, if oxygen is present in the mixture of styrene and the $C_4$-hydrocarbon mixture, the oxygen-containing mixture is first fed to a distillation zone to remove the oxygen, in which zone an oxygen-containing top product and a styrene-containing bottom product are obtained, after which the styrene-containing bottom product is used as the feed for the distillation zone located upstream of the extractive distillation.

6. A process as claimed in claim 5, characterized in that a part of the top product from the distillation zone located upstream of the extractive distillation is fed to the bottom of the distillation zone for the removal of oxygen.

7. A process as claimed in claims 1 to 6, characterized in that the top product from the distillation zone located upstream of the extractive distillation is fed to a butadiene-containing $C_4$-hydrocarbon mixture, which constitutes the main feed stream for the extractive distillation, before being fed to the extractive distillation.

## Revendications

1. Procédé pour isoler le butadiène-1,3 à l'aide d'un solvant sélectif à partir d'un mélange d'hydrocarbures en $C_4$ et de styrène contenant le butadiène-1,3, des hydrocarbures plus solubles que le butadiène-1,3 dans le solvant sélectif et des hydrocarbures moins solubles que le butadiène-1,3 dans le solvant sélectif, et le cas échéant en plus des petites quantités d'oxygène, ce procédé se caractérisant en ce que l'on envoie d'abord le mélange à une zone de distillation dans laquelle on envoie simultanément un deuxième courant d'hydrocarbures liquides ou gazeux, on sépare dans cette zone de distillation, en produit de pied, un mélange d'hydrocarbures en $C_4$ et de styrène et on obtient en produit de tête un mélange d'hydrocarbures en $C_4$, on envoie ensuite le mélange d'hydrocarbures en $C_4$ obtenu en produit de tête de la zone de distillation à une distillation extractive dans laquelle ce mélange d'hydrocarbures en $C_4$ est séparé en un distillat contenant les hydrocarbures moins solubles, un courant de butadiène-1,3 et un courant contenant les hydrocarbures plus solubles.

2. Procédé selon la revendication 1, caractérisé en ce que le deuxième courant envoyé à la zone de distillation consiste en un hydrocarbures saturé à insaturation monooléfinique en $C_4$ ou en un mélange d'hydrocarbures saturés et/ou à insaturation monooléfinique en $C_4$.

3. Procédé selon les revendications 1 et 2, caractérisé en ce que le deuxième courant d'hydrocarbures est introduit dans la zone de distillation au point d'introduction du mélange d'hydrocarbures en $C_4$ et de styrène ou au-dessous de ce point.

4. Procédé selon les revendications 1 à 3, caractérisé en ce que le deuxième courant d'hydrocarbures est introduit dans le tiers inférieur de la zone de distillation.

5. Procédé selon les revendications 1 à 4, caractérisé en ce que, lorsque le mélange d'hydrocarbures en $C_4$ et de styrène contient de l'oxygène, on envoie d'abord ce mélange dans une zone de distillation pour séparation de l'oxygène, dans laquelle on obtient un produit de tête contenant l'oxygène et un produit de pied contenant le styrène, le produit de pied contenant le styrène étant ensuite utilisé comme produit de départ pour la zone de distillation précédant la distillation extractive.

6. Procédé selon la revendication 5, caractérisé en ce que l'on envoie une partie du produit de tête de la zone de distillation précédant la distilla-

|  | Gew.-% |
|---|---|
| Isobuten | 2 |
| Buten-2-trans | 4 |
| Buten-2-cis | 15,67 |
| Butadien-1.3 | 75,0 |
| Butadien-1.2 | 0,2 |
| Butin | 0,1 |
| Vinylcyclohexen | 2,0 |
| Styrol | 1,0 |
| Sauerstoff | 0,03 |

450 g/h dieses Kohlenwasserstoffgemisches werden über Leitung 1 am 4. Boden von unten des Kolonnenoberteils 2 mit 5 Böden eingeleitet. Am Kopf der Kolonne werden 1,6 g/h eines Gemisches aus 12,9 Gew.-% Sauerstoff und Kohlenwasserstoffen über Leitung 13 abgetrennt. Der Kopfdruck wird auf 4,2 bar gehalten. Das flüssige Sumpfprodukt wird in den 5. Boden des Kolonnenunterteils 3 mit 9 Böden eingeleitet. Am Sumpf wird eine Temperatur von 42,7°C festgehalten unter Anpassung der Sumpfabzugsmenge. Durch zusätzliche Zufuhr (über Leitung 5) von Butenen am Kopf der Destillation zur Sauerstoffabtrennung 2 kann der Butadienverlust in dem sauerstoffhaltigen Kopfprodukt dieser Destillation vermindert werden.

Am Kopf des Kolonnenunterteils 3 wird ein Teil des Kohlenwasserstoff-Dampfes von unten in das Oberteil strömen gelassen. ein anderer Teil wird als styrolfreies Produkt mit 0,2 Gew.-ppm Sauerstoff abgezogen (über Leitung 12). Bei Einleiten von 500 g/h eines Butengemisches am Sumpf des Unterteils der Kolonne (über Leitung 11) wird ein Sumpfprodukt mit einem Butadiengehalt von nur 5% erhalten. Das eingesetzte Butengemisch hat folgende Zusammensetzung:

|  | Gew.-% |
|---|---|
| $C_3$-Kohlenwasserstoffe | 0,7 |
| Butan | 13,6 |
| iso-Butan | 3,4 |
| Buten-1 | 26,5 |
| iso-Buten | 39,7 |
| Buten-2-trans | 8,6 |
| Buten-2-cis | 7,2 |
| Butdien-1.3 | 0,2 |
| $C_5$-Kohlenwasserstoffe | 0,1 |

Bei dieser Arbeitsweise beträgt der Butadienverlust im Sumpfprodukt 0,8%.

Werden nur 150 g/h des Butengemisches unter sonst gleichen Bedingungen zugeführt, erhöht sich der Butadienverlust im Sumpfprodukt auf 4,7%. Ohne Zufuhr des Butengemisches beträgt der Butadienverlust im Sumpfprodukt 14,5%, bezogen auf die Butadienmenge im Einsatzprodukt.

Das über Leitung 12 abgezogene styrolfreie $C_4$-Kohlenwasserstoffgemisch wird einer über Leitung 15 zugeführten $C_4$-Fraktion aus einer Äthylenanlage zugemischt, und das erhaltene $C_4$-Kohlenwasserstoffgemisch wird durch extraktive Destillation unter Verwendung von N-Methylenpyrrolidon als selektives Lösungsmittel in der Extraktivdestillationsanlage 9 aufgetrennt.

## Patentansprüche

1. Verfahren zur Gewinnung von Butadien-1,3 mit Hilfe eines selektiven Lösungsmittels aus einer Mischung aus Styrol und einem $C_4$-Kohlenwasserstoffgemisch, welches Butadien-1,3, im selektiven Lösungsmittel löslichere Kohlenwasserstoffe als Butadien-1,3 und im selektiven Lösungsmittel weniger lösliche Kohlenwasserstoffe als Butadien-1,3 enthält, wobei die Mischung gegebenenfalls zusätzlich geringe Mengen Sauerstoff enthält, dadurch gekennzeichnet, dass man die Mischung zunächst einer Destillationszone zuführt, wobei der Destillationszone gleichzeitig ein zweiter Strom von flüssigen oder gasförmigen Kohlenwasserstoffen zugeführt wird, aus der Destillationszone als Sumpfprodukt ein Gemisch aus Styrol und $C_4$-Kohlenwasserstoffen abtrennt und als Kopfprodukt ein $C_4$-Kohlenwasserstoffgemisch erhält und anschliessend das als Kopfprodukt der Destillationszone erhaltene $C_4$-Kohlenwasserstoffgemisch einer extraktiven Destillation zuführt, in der das $C_4$-Kohlenwasserstoffgemisch in ein die weniger löslichen Kohlenwasserstoffe enthaltendes Destillat, einen Strom aus Butadien-1,3 und einen die löslicheren Kohlenwasserstoffe enthaltenden Strom aufgetrennt wird.

2. Verfahren nach Anspruch 1, dadurch gekennzeichnet, dass der Destillationszone als zweiter Strom ein gesättigter oder einfach olefinisch ungesättigter $C_4$-Kohlenwasserstoff bzw. ein Gemisch aus gesättigten und/oder einfach olefinisch ungesättigten $C_4$-Kohlenwasserstoff zugeführt wird.

3. Verfahren nach Anspruch 1 und 2, dadurch gekennzeichnet, dass der zweite Kohlenwasserstoffstrom am oder unterhalb des Zulaufpunktes der Mischung aus Styrol und dem $C_4$-Kohlenwasserstoffgemisch der Destillationszone zugeführt wird.

4. Verfahren nach Anspruch 1 bis 3, dadurch gekennzeichnet, dass der zweite Kohlenwasserstoffstrom im unteren Drittel der Destillationszone zugeführt wird.

5. Verfahren nach Anspruch 1 bis 4, dadurch gekennzeichnet, dass bei Vorhandensein von Sauerstoff in der Mischung aus Styrol und dem $C_4$-Kohlenwasserstoffgemisch die sauerstoffhaltige Mischung zunächst zur Sauerstoffabtrennung einer Destillationszone zugeführt wird, in der ein den Sauerstoff enthaltendes Kopfprodukt und ein das Styrol enthaltendes Sumpfprodukt erhalten werden, und danach das Styrol enthaltende Sumpfprodukt als Einsatzstoff für die der extraktiven Destillation vorgeschaltete Destillationszone verwendet wird.

6. Verfahren nach Anspruch 5, dadurch ge- tion extractive au pied de la zone de distillation pour séparation de l'oxygène.

7. Procédé selon les revendications 1 à 6, caractérisé en ce que le produit de tête de la zone de distillation précédant la distillation extractive est mélangé, avant envoi à la distillation extractive, avec un mélange d'hydrocarbures en $C_4$ contenant du butadiène, qui constitue le courant principal envoyé à la distillation extractive.

FIG.1

FIG.2